# EUROPÄISCHE PATENTANMELDUNG

(11) **EP 2 452 557 A2**
(43) Veröffentlichungstag der Anmeldung: **16.05.2012**
(21) Anmeldenummer: 11008739.2
(22) Anmeldetag: 02.11.2011
(51) Int. Cl.: A01M 1/20

(54) **Wirkstoff-Abgabevorrichtung**

(30) Priorität: 16.11.2010 DE 102010051409
(71) Anmelder: Budich International GmbH, 32120 Hiddenhausen (DE)
(72) Erfinder: Budich, Meinrad, 32609 Hüllhorst (DE)
(74) Vertreter: Beckord & Niedlich

(57) **Zusammenfassung**

Die Erfindung betrifft eine Wirkstoff-Abgabevorrichtung (1) zur Abgabe von gasförmigen Wirkstoffen, insbesondere von Duftstoffen, in die Umgebung: Die Wirkstoff-Abgabevorrichtung (1) weist ein Gehäuse (3) und einen dem Gehäuse (3) zugeordneten Wirkstoffträger (2, 2') auf. Das Gehäuse (3) umfasst ein Gehäuseunterteil (3a) und ein Gehäuseoberteil (3b), wobei das Gehäuseoberteil (3b) und das Gehäuseunterteil (3a) unter Bildung eines Spalts (17) mit einem Abstand (a) voneinander angeordnet sind. In dem Spalt (17) ist zumindest teilweise der Wirkstoffträger (2, 2') angeordnet. Darüber hinaus betrifft die Erfindung ein Gehäuse (3), einen Wirkstoffträger (2, 2') sowie einen Wirkstoffbehälter (30) für eine derartige Wirkstoff-Abgabevorrichtung (1).

## Beschreibung

Die Erfindung betrifft eine Wirkstoff-Abgabevorrichtung zur Abgabe von gasförmigen Wirkstoffen, insbesondere Duftstoffen, in die Umgebung. Diese Wirkstoff-Abgabevorrichtung weist ein Gehäuse und einen dem Gehäuse zugeordneten Wirkstoffträger auf. Darüber hinaus betrifft die Erfindung ein entsprechendes Gehäuse sowie einen entsprechenden Wirkstoffträger und einen in den Wirkstoffträger einsetzbaren Wirkstoffbehälter.

Wirkstoff-Abgabevorrichtungen der eingangs genannten Art sind in ganz unterschiedlichen Ausgestaltungen aus dem Stand der Technik bekannt. Sie dienen dazu, die unmittelbare Umgebung, beispielsweise die Atmosphäre in einem Raum, zu parfümieren und/oder zu desodorieren. Es sind Duftspender bekannt, bei denen ein flüssiger Duftstoff in einer Flasche aufgenommen ist, deren Öffnung mit einer Kappe verschlossen ist. Der Duftspender besitzt einen an einem Dochthalter befestigten Docht, der von der Öffnung aus in die Flasche hineinragt und der beim Gebrauch des Duftspenders teilweise in den Duftstoff eintaucht und teilweise aus dem Duftstoff herausragt. Somit dringt der flüssige Duftstoff in den Docht ein, stiegt in diesem nach oben und dampft vom Docht ab. Die Abdampfrate (d. h., wie stark der Duftspender den flüssigen Duftstoff in die Umgebung abgibt) lässt sich auf verschiedene Weise einstellen. Bei einigen Vorrichtungen geschieht dies dadurch, dass eine Verschlusskappe mehr oder weniger weit von der Flasche abgeschraubt wird. Bei anderen Duftspendern wird die Abdampfrate dadurch eingestellt, dass der Docht mehr oder weniger hoch aus der Flasche herausgezogen wird.

Eine weitere Wirkstoff-Abgabevorrichtung wird in der DE 297 00 718 U1 beschrieben. Diese besteht aus einem Behälter mit zwei ineinander angeordneten Kammern. In einer inneren, ersten Kammer befindet sich vor der Inbetriebnahme ein flüssiger Wirkstoff. In einer äußeren, zweiten Kammer befindet sich ein Block aus kristallinem Trägerstoff. Bei Inbetriebnahme der Wirkstoff-Abgabevorrichtung wird dafür gesorgt, dass die Flüssigkeit von der ersten in die zweite Kammer strömen kann. Bei Kontakt der Flüssigkeit mit dem Trägerstoff löst sich der Block auf und der Trägerstoff verbindet sich mit der Flüssigkeit zu einem Gel, das die zweite Kammer ausfüllt. Durch einen oberhalb des Gels befindlichen Spalt in der äußeren Kammer kann der Duftstoff dann nach und nach entweichen.

Es ist Aufgabe der Erfindung, eine alternative Wirkstoff-Abgabevorrichtung zur Abgabe von gasförmigen Wirkstoffen, insbesondere Duftstoffen, in die Umgebung bereitzustellen.

Die Aufgabe wird zum einen durch eine Wirkstoff-Abgabevorrichtung nach Patentanspruch 1 und zum anderen durch ein Gehäuse nach Anspruch 13 sowie durch einen Wirkstoffträger nach Anspruch 14 und einen Wirkstoffbehälter nach Anspruch 15 gelöst.

Die erfindungsgemäße Wirkstoff-Abgabevorrichtung zur Abgabe von gasförmigen Wirkstoffen, insbesondere Duftstoffen, in die Umgebung, weist ein Gehäuse und ein dem Gehäuse zugeordneten Wirkstoffträger auf, der einen die Wirk- bzw. Duftstoffe abgebenden Trägerstoff aufnehmen kann. Das Gehäuse ist aus einem Gehäuseunterteil und einem Gehäuseoberteil gebildet. Dabei kann das Gehäuseunterteil eine Standfläche aufweisen, die eine kippsichere Platzierung der Wirkstoff-Abgabevorrichtung auf einer ebenen Fläche, z. B. einem Tisch, ermöglicht, während das Gehäuseoberteil als eine Art Abdeckung ausgebildet sein kann. Das Gehäuseoberteil und das Gehäuseunterteil des Gehäuses sind so zueinander angeordnet, dass sie einen Spalt mit einem Abstand, vorzugsweise einem nicht weiter reduzierbaren Mindestabstand bilden. In diesem vorzugsweise sich in einer vertikalen Ebene erstreckenden, radial nach außen offenen Spalt ist zumindest teilweise der Wirkstoffträger angeordnet.

Die Wirkstoff-Abgabevorrichtung ist einfach aufgebaut und besteht nur aus wenigen Teilen. Der Wirkstoffträger ist auf einfache Weise bei der Montage der Wirkstoff-Abgabevorrichtung einfach montierbar und später werkzeuglos leicht entnehmbar bzw. austauschbar oder neu befüllbar, wenn der Wirkstoffvorrat erschöpft ist. Der Wirkstoffträger, bei dem es sich um ein mechanisches Vorrichtungsteil handelt, ist vorzugsweise scheibenförmig ausgebildet. In oder an diesem Wirkstoffträger ist, wie später noch genauer erläutert wird, direkt der Wirkstoff gegebenenfalls mitsamt einem Trägerstoff bzw. Trägermaterial für den Wirkstoff untergebracht, oder er nimmt einen Wirkstoffbehälter auf, in dem sich der Wirkstoff eventuell mit einem Trägerstoff befindet.

Die abhängigen Ansprüche und die weitere Beschreibung enthalten besonders vorteilhafte Ausgestaltungen und Weiterbildungen der Erfindung, wobei insbesondere die Ansprüche einer Kategorie auch analog den Ansprüchen einer der anderen Kategorien weitergebildet sein können.

Ein besonders simpler Aufbau ergibt sich, wenn der Wirkstoffträger ausschließlich in dem Spalt zwischen dem Gehäuseoberteil und dem Gehäuseunterteil angeordnet ist oder sich nur geringfügig in das Gehäuseunterteil und/oder Gehäuseoberteil erstreckt. Dies erlaubt einen besonders einfachen Wechsel des Wirkstoffträgers.

Vorzugsweise sind das Gehäuseoberteil und das Gehäuseunterteil sowie der Wirkstoffträger in zumindest einer ersten Position (Ausgangsposition) so angeordnet, dass eine Gehäuseoberteilkontaktfläche des Gehäuseoberteils an eine erste Wirkstoffträgerkontaktfläche des Wirkstoffträgers angrenzt, besonders bevorzugt mit dieser in Kontakt steht. Dabei grenzt bevorzugt auch eine Gehäuseunterteilkontaktfläche des Gehäuseunterteils an eine zweite Wirkstoffträgerkontaktfläche des Wirkstoffträgers an, bzw. steht besonders bevorzugt mit dieser in Kontakt. Somit weist die Wirkstoff-Abgabevorrichtung in dieser ersten Position einen sandwichartigen Aufbau mit einem zwischen dem Gehäuseoberteil und dem Gehäuseunterteil angeordneten Wirkstoffträger auf. Dabei kann die Wirkstoff-Abgabevorrichtung eine in dieser ersten Position unverschlossene Ausdampföffnung in den Gehäuseteilen und/oder dem Wirkstoffträger zum Austritt der gasförmigen Wirkstoffe aufweisen, um eine Mindestabdampfrate zu gewährleisten. Diese Ausdampföffnung kann beispielsweise durch eine Anzahl von (d.h. ein oder mehrere) Luftströmungslöchern bzw. Aussparungen im Gehäuseoberteil und/oder im Gehäuseunterteil und/oder im Wirkstoffträger gebildet sein.

Wenn die Wirkstoff-Abgabevorrichtung (gegebenenfalls auch erst durch eine nachfolgend erläuterte weitere Öffnung des Spaltes) mehrere Ausdampföffnungen bzw. Luftströmungslöcher an entsprechenden Positionen aufweist, insbesondere auf unterschiedlichen Höhen relativ zu einer Standfläche, kann hierdurch eine Art "Kamineffekt" entstehen. Dabei kann dann Luft z.B. durch eine Anzahl von weiter unten liegenden Öffnungen in das Gehäuse einströmen, am Wirkstoffträger vorbei und durch eine Anzahl von weiter oben liegende Öffnungen wieder ausströmen. Dadurch kann eine besonders gute Verteilung des Wirkstoffs erreicht werden.

Um die Abdampfrate bei Bedarf erhöhen zu können, kann der Abstand vom Gehäuseoberteil zu dem Gehäuseunterteil durch Verlagern des Gehäuseoberteils relativ zum Gehäuseunterteil entlang einer Verbindungsachse variiert werden, die zwischen Gehäuseunterteil und Gehäuseoberteil verläuft und das Gehäuseoberteil mit dem Gehäuseunterteil verbindet. Hierdurch wird die Breite des Spaltes entlang der Verbindungsachse erhöht und damit die Gesamtfläche der Ausdampföffnung vergrößert. Es sind also das Gehäuseoberteil und das Gehäuseunterteil derart miteinander verbunden, dass ausgehend von einer ersten Position bzw. Ausgangsposition ein Spalt mit einer Mindestbreite vergrößert werden kann.

Hierzu können das Gehäuseunterteil und das Gehäuseoberteil vorzugsweise derart miteinander durch eine Gehäuseführung gekoppelt sein, dass eine lineare Bewegung des Gehäuseoberteils relativ zum Gehäuseunterteil entlang der Verbindungsachse durchführbar ist. Dies bewirkt, dass der Spalt bei einem Verlagern des Gehäuseoberteils in Bezug zum Gehäuseunterteil an all seinen Positionen gleichmäßig vergrößert wird, d. h. die Kontaktflächen der Gehäuseteile und des Wirkstoffträgers bleiben trotz der Verlagerung parallel zueinander. In einer alternativen Ausführungsform können das Gehäuseunterteil und das Gehäuseoberteil so miteinander gekoppelt sein, dass eine Bewegung um eine Drehachse durchgeführt wird, d. h. das Gehäuseoberteil wird in Bezug zum Gehäuseunterteil um eine Achse verschwenkt, so dass sich das Gehäuse ähnlich wie eine Muschel öffnet und aufgrund dieser Schwenkbewegung der Spalt an verschiedenen Stellen unterschiedlich stark vergrößert wird. In diesem Fall werden die Kontaktflächen durch das Verlagern aus ihrer parallelen Lage gebracht. Die Gehäuseführung kann einen Anschlag aufweisen, der ein Unterschreiten eines Mindestabstands durch z. B ein Weiterdrehen verhindert.

Wenn die Gehäuseführung derart ausgebildet ist, dass eine lineare Bewegung des Gehäuseunterteils in Bezug zum Gehäuseoberteil durchführbar ist, ist eine besonders einfache Bedienung, d. h. Veränderung der Position des Gehäuseoberteils in Bezug zum Gehäuseunterteil möglich, wenn das Gehäuseunterteil und das Gehäuseoberteil so gekoppelt sind, dass eine Drehbewegung des Gehäuseoberteils und/oder Gehäuseunterteils um die Verbindungsachse zwangsläufig in eine lineare Bewegung des Gehäuseoberteils relativ zum Gehäuseunterteil entlang der Verbindungsachse umgewandelt wird. Hierdurch kann eine Bedienperson durch Drehen des Gehäuseoberteils und gleichzeitiges Festhalten des Gehäuseunterteils eine Vergrößerung des Spalts bewirken, wenn dies gewünscht ist. Dabei erlaubt eine derartige Kopplung des Gehäuseunterteils mit dem Gehäuseoberteil eine besonders genaue Einstellung der Breite des gewünschten Spaltes. Hierzu kann die Kopplung derart ausgebildet sein, dass eine Drehbewegung in eine kontinuierliche lineare Bewegung entlang der Verbindungsachse umgewandelt wird. Z. B. kann die Gehäuseführung mit einem ersten Gewindeabschnitt versehen sein, der in einen zweiten Gewindeabschnitt der Gehäuseführung eingreift. In einer alternativen Ausführungsform können Stifte bzw. Dorne, die einem Gehäuseteil zugeordnet sind, in eine Schlitze aufweisende Kulisse eingreifen und so bewirken, dass eine Drehbewegung in eine lineare Bewegung umgesetzt wird. So kann ebenfalls eine kontinuierliche Umsetzung einer Drehbewegung in eine lineare Bewegung bewirkt werden. Es ist aber auch möglich, durch Gestaltung der Schlitze eine stufenförmige Umsetzung einer Drehbewegung in eine lineare Bewegung zu bewirken. Dabei können zusätzlich Rastmittel vorgesehen sein, um eine Fixierung des Gehäuseoberteils in der gewünschten Position sicherzustellen.

Das Einsetzen eines Wirkstoffträgers in das Gehäuse bzw. das Auswechseln eines Wirkstoffträgers oder eines Wirkstoffbehälters im Wirkstoffträger nach Erschöpfung des gasförmigen Wirkstoffs ist durch ein Verlagern des Wirkstoffträgers relativ zum Gehäuse quer zur Verbindungsachse möglich. Im einfachsten Fall wird also der Wirkstoffträger entlang einer Montageachse bewegt, die senkrecht zur Verbindungsachse des Gehäuseunterteils und des Gehäuseoberteils verläuft. Dies erlaubt ein besonders einfaches Einsetzen bzw. einen Austausch des Wirkstoffträgers bzw. Wirkstoffbehälters. Alternativ ist es auch möglich, das Gehäuseoberteil vollständig vom Gehäuseunterteil zu trennen, und den Wirkstoffträger durch eine Bewegung entlang der Verbindungsachse des Gehäuseunterteils und des Gehäuseoberteils in das Gehäuse einzufügen und anschließend das Gehäuseunterteil mit dem Gehäuseoberteil wieder zu verbinden.

Es kann nur ein Wirkstoffträger zwischen dem Gehäuseunterteil und dem Gehäuseoberteil angeordnet sein. Es ist aber auch möglich, dass mehrere Wirkstoffträger mit unterschiedlichen gasförmigen Wirkstoffen, insbesondere unterschiedlichen Duftnoten, zwischen oder im Gehäuseunterteil und/oder im Gehäuseoberteil angeordnet werden, um so verschiedene Duftnoten auf Wunsch miteinander kombinieren zu können.

Zur Aufnahme des Wirkstoffs weist der Wirkstoffträger vorzugsweise eine Kavität auf. Dabei kann der Wirkstoffträger mitsamt der Kavität beispielsweise durch Tiefziehen von Kunststoff oder durch Spritzguss gefertigt werden.

Der Wirkstoff selbst kann in einem Trägermaterial bzw. Trägerstoff wie einem Vlies , sonstigem geeigneten Fasermaterial oder einem anderen porösen Material aufgenommen sein. Die Kavität kann z. B. mittels einer gaspermeablen (semipermeablen) Membran verschlossen sein, die nur einen gasförmigen Wirkstoff hindurch treten lässt, ansonsten aber z. B. einen ungewünschten Eintritt von Flüssigkeiten in die Kavität oder einen Austritt einer Wirkstoffträgerflüssigkeit aus der Kavität verhindert. Insbesondere in diesem Fall kann also auch ein flüssiger Trägerstoff bzw. verflüssigter Wirkstoff eingesetzt werden. Ebenso ist es möglich, die Kavität mit einem bereits gasförmigen Wirkstoff zu füllen, wobei die Kavität dann durch eine Membran verschlossen ist, die nur eine bestimmte Durchlassrate für den Wirkstoff erlaubt.

Bei einer bevorzugten Variante ist in der Kavität ein gelartiger Trägerstoff aufgenommen, der den gasförmigen Wirkstoff, z. B. Duftstoffe, freisetzt. Dies erlaubt eine besonders einfache Handhabung des Wirkstoffträgers, da durch den gelartigen Trägerstoff ein unbeabsichtigter Austritt des Wirkstoffs, insbesondere beim Einsetzen des Wirkstoffträgers in den Spalt des Gehäuses, ausgeschlossen ist. In diesem Fall könnte insbesondere darauf verzichtet werden, dass die Kavität mit einer semipermeablen Membran verschlossen ist, wobei die Verwendung einer solchen Membran aber auch bei einem Gel nicht ausgeschlossen ist.

Bei einer weiteren bevorzugten Variante wird am Wirkstoffträger, besonders bevorzugt in der Kavität, ein mit dem Wirkstoff und gegebenenfalls dem Trägermaterial gefüllter Wirkstoffbehälter angeordnet. Dies hat den Vorteil, dass bei verbrauchtem Wirkstoff nur der Wirkstoffbehälter ausgewechselt werden muss, was Ressourcen schonender und kostengünstiger ist.

Dabei kann vorzugsweise auch der Wirkstoffbehälter mit einer gasdurchlässigen Membran verschlossen sein und zum Beispiel mit einem flüssigen oder gasförmigen Wirkstoff bzw. Trägerstoff gefüllt sein. Ebenso kann aber der Wirkstoffbehälter auch offen sein und z.B. mit einem wirkstoffhaltigen Gel oder dergl. gefüllt sein.

Der Wirkstoffbehälter kann relativ dünnwandig sein, vorzugsweise aus Tiefziehfolie hergestellt werden. Der Wirkstoffträger kann auch mehrere Kammern mit verschiedenen Wirkstoffen, z.B. unterschiedlichen Duftnoten, umfassen.

Die Außenmaße des Wirkstoffbehälters sind vorzugsweise an die Kavität angepasst. Besonders bevorzugt befinden sich am Wirkstoffträger, insbesondere in der Kavität des Wirkstoffträgers, und/oder am Wirkstoffbehälter Halteelemente, durch die der Wirkstoffbehälter gut am Wirkstoffträger, z.B. in der Kavität, festgehalten wird. Besonders bevorzugt weisen sowohl der Wirkstoffträger als auch der Wirkstoffbehälter zusammenwirkende Halteelemente auf. Beispielsweise kann in der Kavität des Wirkstoffträgers ein nach innen ragender Vorsprung, insbesondere eine Wulst oder dergleichen, angeordnet sein, um eine Klemmung des Wirkstoffbehälters in der Kavität zu erreichen. Am Wirkstoffbehälter selber kann ein hierzu passender Vorsprung oder eine passende Ausnehmung, beispielsweise eine Sicke, angeordnet sein, welche mit dem Vorsprung in der Kavität zusammen wirkt. Umgekehrt kann auch die Kavität eine Ausnehmung aufweisen, die mit einem Vorsprung am Wirkstoffbehälter zusammen wirkt.

Um eine besonders einfache Handhabung und Lagerung von Wirkstoffträgern zu ermöglichen, ohne dass es zu einem vorzeitigen Austritt der Wirkstoffe kommt, sind die Kavität oder der Wirkstoffbehältern vor Inbetriebnahme gasundurchlässig verschlossen, z. B. mittels eines gasundurchlässigen entfernbaren Schutzdeckels, der gegebenenfalls wiederum die gasdurchlässige Membran abdeckt. Dabei kann der gasundurchlässige Schutzdeckel als Folie ausgebildet sein, die z. B. mittels einer lösbaren Klebeverbindung am Wirkstoffträger derart angebracht ist, dass die Kavität bzw. der Wirkstoffbehälter gasundurchlässig verschlossen ist. Anstelle der Klebeverbindung kann auch eine Press- oder Rastverbindung vorgesehen sein, mit der z. B. ein im Vergleich zu einer Folie steiferer Verschlussdeckel die Kavität verschließt.

Das Gehäuseoberteil und das Gehäuseunterteil sowie der Wirkstoffträger und/oder der Wirkstoffbehälter können im Prinzip eine beliebige äußere Form aufweisen. Eine besonders optisch ansprechende Gestaltung des Gehäuses ergibt sich, wenn das Gehäuseunterteil und das Gehäuseoberteil jeweils angrenzend an den Spalt senkrecht zur Längsrichtung der Verbindungsachse einen kreisförmigen Außen-Querschnitt aufweisen. Dabei weisen die kreisförmigen Außen-Querschnitte jeweils den gleichen Durchmesser auf. Durch diese Gestaltung des Gehäuseunterteils, des Gehäuseoberteils und des Wirkstoffträgers wird insbesondere eine im Wesentlichen rotationssymmetrische Gestaltung, z. B. Kugelform, ohne unästhetische Formabweichung, wie z. B. Stufen, möglich, auch wenn der Spalt durch eine Drehbewegung des Gehäuseoberteils in Bezug zum Gehäuseunterteil vergrößert wird.

Die optimale Wahl der Maße des Gehäuses und des Wirkstoffträgers hängt vom Einsatzzweck und -ort, von der Art des Wirkstoffs, von der gewählten Wirkstoffrate und Wirkstoffmenge als auch von der gewünschten Ästhetik ab. Vorzugsweise sind das Gehäuse und der Wirkstoffträger so dimensioniert, dass der Durchmesser des Außen-Querschnitts der Gehäuseteile angrenzend an den Spalt senkrecht zur Längsrichtung der Verbindungsachse sowie der Außen-Durchmesser des Wirkstoffträgers jeweils zwischen 5 cm und 20 cm, besonders bevorzugt zwischen 7 cm und 12 cm beträgt. Die Dicke (bzw. Höhe) des scheibenförmigen Wirkstoffträgers kann vorzugsweise 2 mm bis 7 mm betragen, wobei die Füllhöhe in der Kavität vorzugsweise zwischen 1 mm und 6 mm beträgt. Die Kopplung und Ausbildung der Gehäuseteile ist vorzugsweise so, dass die mögliche Maximalbreite des Spalts, d.h. bei ganz hochgefahrenem oberem Gehäuseteil, zwischen 5 mm und 25 mm liegt. Vorzugsweise beträgt in dieser Stellung die Öffnung des umlaufenden Spalts oberhalb des eingesetzten Wirkstoffträger bis zu 20 mm und mehr.

Um das Austauschen eines neuen Wirkstoffträgers oder eines darin angeordneten Wirkstoffbehälters nach Verbrauch des Wirkstoffs zu erleichtern, ist eine Wirkstoffträgerführung vorgesehen. Sie führt den Wirkstoffträger beim Einschieben in den Spalt und weist ferner einen Anschlag auf, um diese Einführbewegung zu stoppen. Dabei ergibt sich ein besonders einfacher Aufbau, wenn diese Wirkstoffträgerführung und dieser Anschlag mit der Gehäuseführung des Gehäuses zusammenwirken, die eine Verlagerung des Gehäuseunterteils und des Gehäuseoberteils relativ zueinander erlauben. Das heißt, dass die Gehäuseführung eine Doppelfunktion übernimmt, nämlich zum einen eine Führung des Gehäuseoberteils in Bezug zum Gehäuseunterteil entlang der Verbindungsachse und zum anderen, zusammen mit der Wirkstoffträgerführung, eine Führung des Wirkstoffträgers entlang einer Montageachse bei einem Einschieben des Wirkstoffträgers in den Spalt, z. B. senkrecht zur Verbindungsachse.

Eine besonders ästhetische Gestaltung des Gehäuses wird erreicht, wenn die Gehäuseführung, z.B. die Verbindungsachse, im Inneren Gehäuseunterteils und/oder des Gehäuseoberteils angeordnet ist. Somit ist diese Gehäuseführung im Inneren des Gehäuses für eine Bedienperson unsichtbar verborgen. Die Gehäuseführung kann aber auch außerhalb des Gehäuseunterteils und/oder des Gehäuseoberteils angeordnet sein.

Eine besonders materialsparende und leichte Ausbildung des Gehäuseunterteils und/oder des Gehäuseoberteils kann erreicht werden, wenn das Gehäuseunterteil und/oder das Gehäuseoberteil als offene Hohlkörper ausgebildet sind. Zum Beispiel können das Gehäuseunterteil und/oder das Gehäuseoberteil kalottenförmig ausgebildet sein bzw. die Gestalt von hohlen Halbkugeln aufweisen. Somit weisen das Gehäuseunterteil und das Gehäuseoberteil einen Innenraum auf, in dem dann die Gehäuseführung, bzw. die Verbindungsachse, angeordnet sein kann. Dies erlaubt ferner eine besonders einfache Fertigung des Gehäuseunterteils und des Gehäuseoberteils aus Kunststoff mittels Spritzguss.

Die Ausgestaltung eines zum Wirkstoff im Wirkstoffträger weisenden Gehäuseteils, insbesondere des Gehäuseoberteils, als Hohlraum, hat den Vorteil, dass dieser als eine Art "Mischraum" dienen kann, in dem sich die Wirkstoffe ausbreiten und mit Luft vermischen können. Somit wirkt eine Vergrößerung des Spalts relativ schnell.

Wenn das Gehäuseunterteil vollständig vom Gehäuseoberteil getrennt ist, z.B. nach der Herstellung der einzelnen Teile, ist eine besonders einfache Montage durch Zusammenführen des Gehäuseunterteils mit dem Gehäuseoberteil sowie einem oder mehreren Wirkstoffträgern möglich, wenn die Gehäuseführung zweigeteilt ausgebildet ist, d. h. einen ersten Abschnitt und einen zweiten Abschnitt aufweist. Dabei kann der erste Abschnitt z. B. dem Gehäuseunterteil und der zweite Abschnitt z. B. dem Gehäuseoberteil zugeordnet sein. Die Länge des ersten und/oder zweiten Abschnitts kann derart gewählt sein, dass sie sich in Richtung der Verbindungsachse aus dem Gehäuseunterteil und/oder Gehäuseoberteil erstrecken. Wenn z. B. das Gehäuseunterteil und/oder das Gehäuseoberteil als hohle Halbkugeln ausgebildet sind, ist in diesem Fall die Länge des ersten und/oder zweiten Abschnitts größer als der Radius der Halbkugel. Dieses Vorstehen des ersten und des zweiten Abschnitts vereinfacht dann das Zusammenfügen des Gehäuseunterteils und des Gehäuseoberteils und ermöglicht den gewünschten Spalt zwischen den Gehäuseteilen.

Die Erfindung wird im Folgenden unter Hinweis auf die beigefügten Figuren anhand von Ausführungsbeispielen noch einmal näher erläutert. Hieraus ergeben sich auch weitere Einzelheiten und Vorteile der Erfindung. Es zeigen:
- Figur.1: eine perspektivische Darstellung einer erfindungsgemäßen Wirkstoff-Abgabevorrichtung gemäß einem ersten Ausführungsbeispiel,
- Figur 2: eine perspektivische Ansicht einer erfindungsgemäßen Wirkstoff-Abgabevorrichtung mit einer vergrößerter Ausdampföffnung gemäß dem ersten Ausführungsbeispiel,
- Figur 3: eine weitere perspektivische Darstellung einer erfindungsgemäßen Wirkstoff-Abgabevorrichtung mit einer vergrößerten Ausdampföffnung gemäß dem ersten Ausführungsbeispiel,
- Figur 4: eine perspektivische Darstellung eines Gehäuseoberteils des Gehäuses einer Wirkstoff-Abgabevorrichtung gemäß dem ersten Ausführungsbeispiel,
- Figur 5: eine perspektivische Darstellung eines Gehäuseunterteils eines Gehäuses der Wirkstoff-Abgabevorrichtung gemäß dem ersten Ausführungsbeispiel,
- Figur 6: eine perspektivische Darstellung eines Wirkstoffträgers gemäß einem ersten Ausführungsbeispiel,
- Figur 7: eine Explosionsdarstellung einer erfindungsgemäßen Wirkstoff-Abgabevorrichtung gemäß einem zweiten Ausführungsbeispiel,
- Figur 8: eine Draufsicht und Seitenansicht des Gehäuseunterteils des Gehäuses der Wirkstoff-Abgabevorrichtung gemäß dem zweiten Ausführungsbeispiel,
- Figur 9: eine Draufsicht und Seitenansicht eines Gehäuseoberteils des Gehäuses der Wirkstoff-Abgabevorrichtung gemäß dem zweiten Ausführungsbeispiel,
- Figur 10: eine Draufsicht und Seitenansicht des Wirkstoffträgers gemäß Figur 6, und
- Figur 11: eine perspektivische Darstellung einer Wirkstoff-Abgabevorrichtung gemäß einem dritten Ausführungsbeispiel,
- Figur 12: eine Draufsicht und Seitenansichten (mit und ohne Wirkstoffbehälter) eines Wirkstoffträgers gemäß einem zweiten Ausführungsbeispiel,
- Figur 13: eine Draufsicht und eine Seitenansicht eines Wirkstoffbehälters für einen Wirkstoffträger gemäß Figur 12.

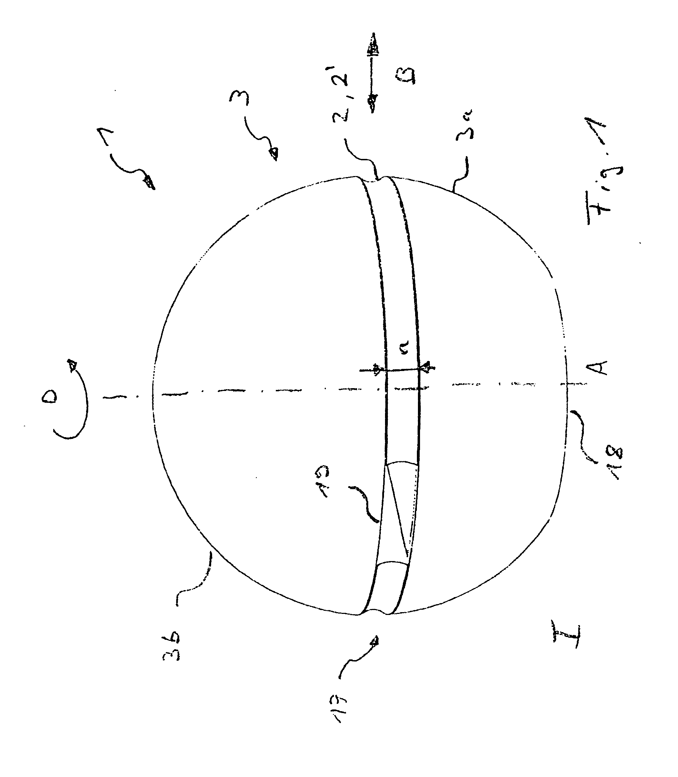

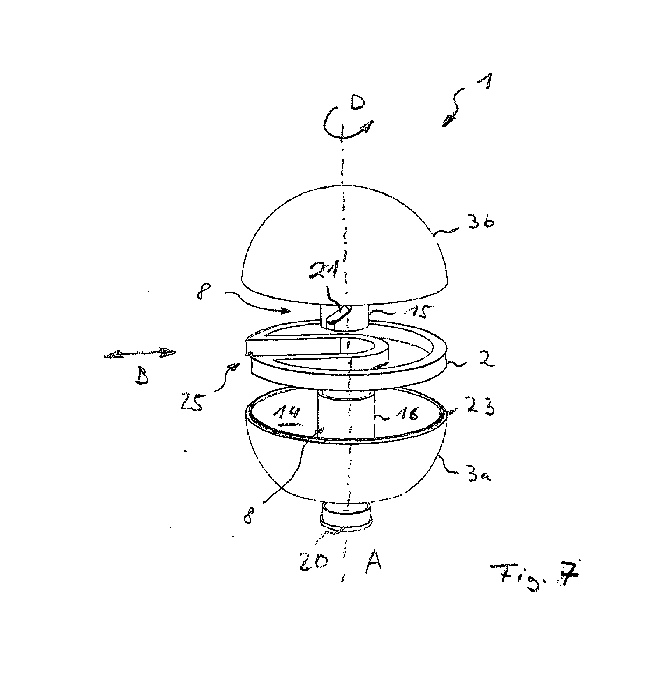

Es wird zunächst auf die Figuren 1 bis 3 sowie auf Figur 7 Bezug genommen.

Die Wirkstoff-Abgabevorrichtung 1 besteht aus einem Gehäuse 3, das aus einem Gehäuseunterteil 3a und einem Gehäuseoberteil 3b gebildet ist. Das Gehäuseunterteil 3a weist eine Standfläche 18 auf, mit der die Wirkstoff-Abgabevorrichtung 1 auf einer flachen Fläche, wie z. B. einer Tischplatte, abgestellt werden kann. Das Gehäuseunterteil 3a und das Gehäuseoberteil 3b sind außenseitig halbkugelförmig ausgebildet, so dass zusammengefügt das Gehäuse 3 eine nahezu kugelförmige Gestalt bis auf die Abflachung zur Bildung der Standfläche 18. aufweist.

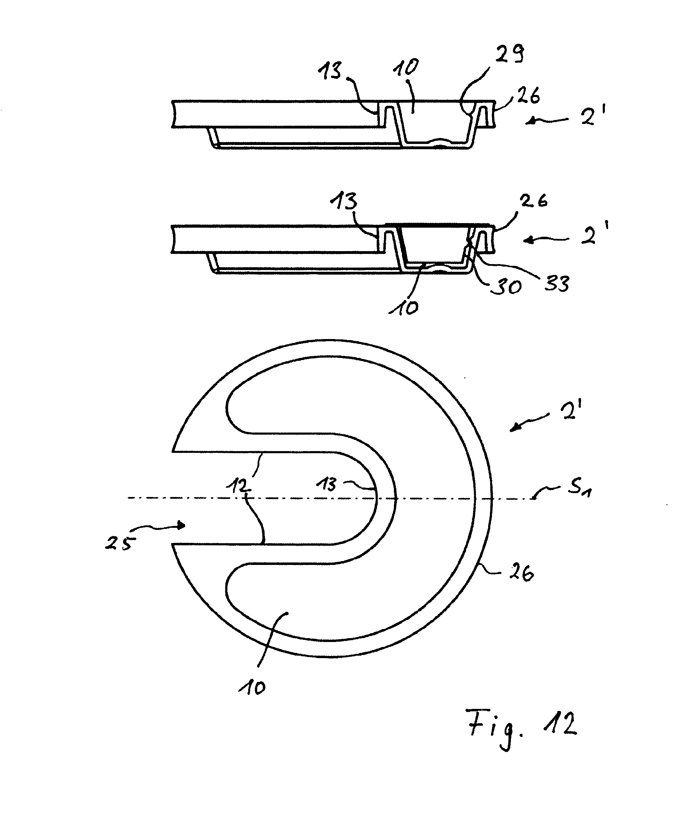

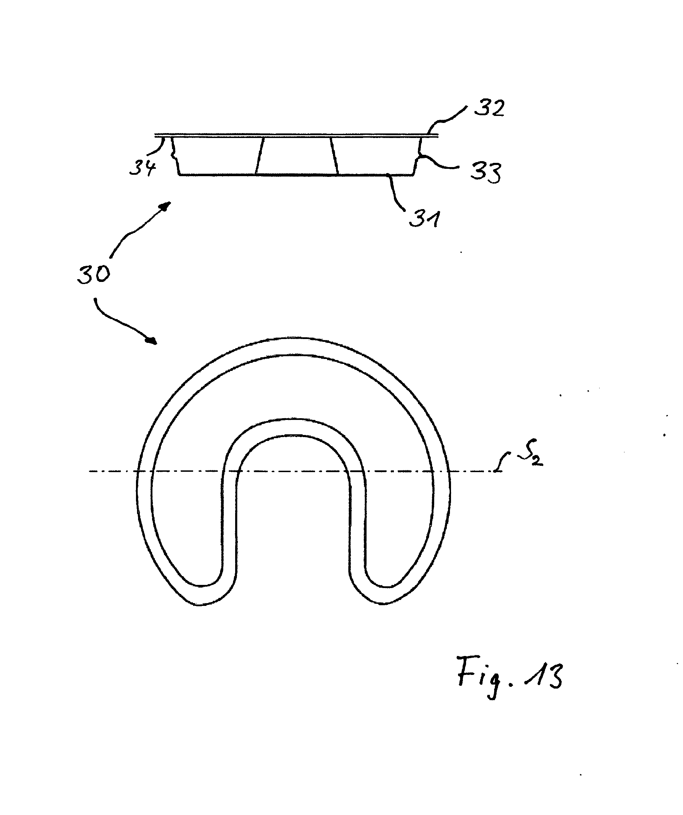

Das Gehäuseoberteil 3b und das Gehäuseunterteil 3a sind in einer Ausgangsposition I mit einem Abstand a voneinander angeordnet, so dass zwischen dem Gehäuseunterteil 3a und dem Gehäuseoberteil 3b ein Spalt 17 gebildet ist. In diesem Spalt 17 ist ein Wirkstoffträger 2, 2' eingesetzt. Der Wirkstoffträger 2 weist im vorliegenden Ausführungsbeispiel einen gelförmigen Trägerstoff 27 (siehe Figur 7) auf, der z. B. Duftstoffe an die Raumluft abgeben kann. Alternativ kann es sich um einen Wirkstoffträger 2' mit einem darin angeordneten Wirkstoffbehälter 30 handeln, wie er später noch anhand der Figuren 12 und 13 näher erläutert wird.

Die gasförmigen Wirkstoffe können in der in Figur 1 gezeigten Ausgangsposition I aus einer durch eine Aussparung im Wirkstoffträger 2 gebildete Ausdampföffnung 19 entweichen, die von Abschnitten des Wirkstoffträgers 2 des Gehäuseunterteils 3a und des Gehäuseoberteils 3b begrenzt wird.

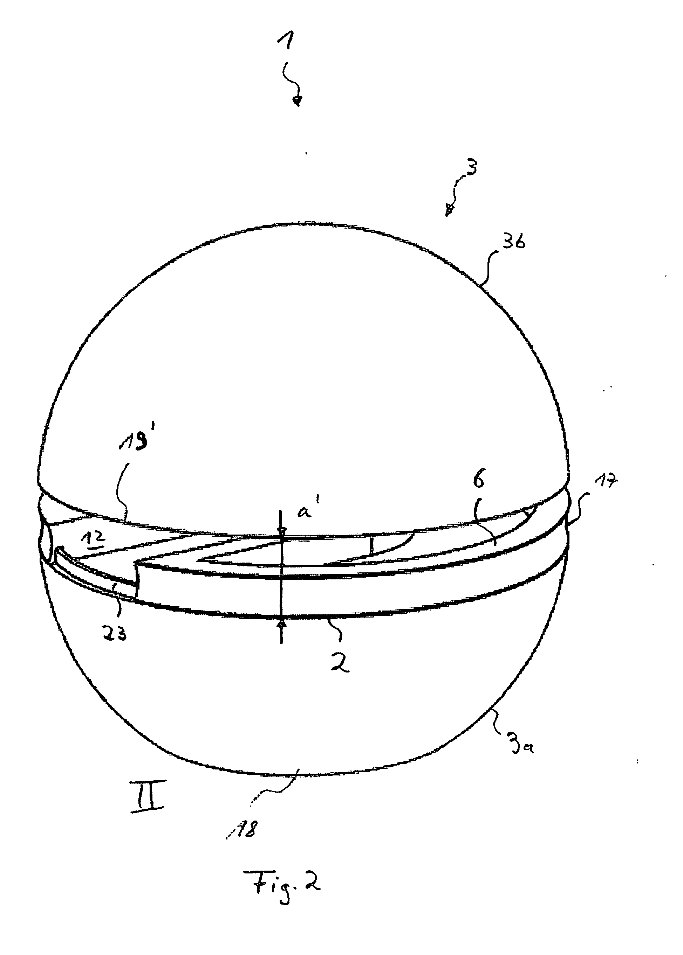

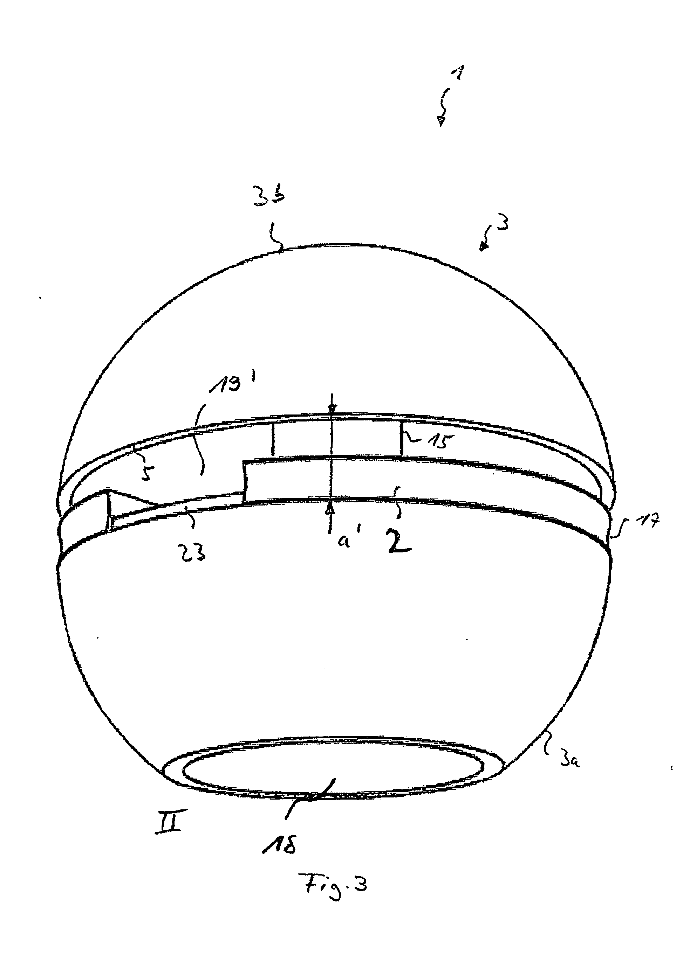

Um die Abdampfrate zu erhöhen, kann die Ausdampföffnung 19 vergrößert werden. Hierzu wird das Gehäuseoberteil 3b in eine - in den Figuren 2 und 3 dargestellte - zweite Position II mit einem vergrößerten Abstand a' gebracht, in der der Spalt 17 vergrößert ist und nunmehr die Ausdampföffnung 19' um einen ringförmigen Abschnitt erweitert ist. Um das Gehäuseoberteil 3b aus der Ausgangsposition I in die Position II zu bringen, wird das Gehäuseoberteil 3b entlang einer Verbindungsachse A (Figur 1) verlagert.

Sowohl das Gehäuseunterteil 3a als auch das Gehäuseoberteil 3b sind als Hohlkörper bzw. kalottenförmig ausgebildet, d. h. das Innere des Gehäuses 3 ist hohl. Dies erlaubt es, dass sich die Wirkstoffe im Inneren des Gehäuses 3 ausbreiten können und auch in der geschlossenen Position I mit Luft vermischen können. Somit steht bei einer Vergrößerung des Spalts 17 sehr schnell, fast verzögerungsfrei, ein besonders intensives Dufterlebnis zur Verfügung, wenn es sich bei den gasförmigen Wirkstoffen um Duftstoffe handelt.

Zugleich ist in dieser Position II ein Wechsel des Wirkstoffträgers 2 möglich, wenn der Vorrat an gasförmigen Wirkstoffen sich erschöpft hat. Hierzu wird der Wirkstoffträger 2 zeitweise entlang einer Montageachse B bewegt, die senkrecht zu der Verbindungsachse A verläuft.

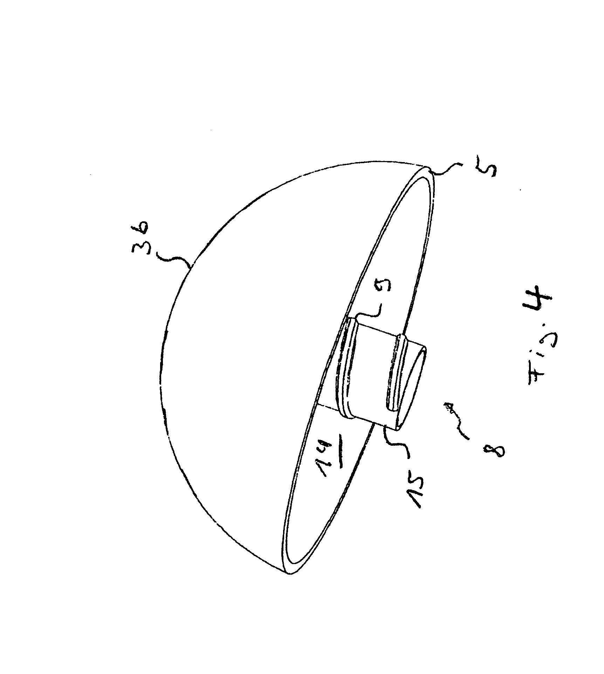

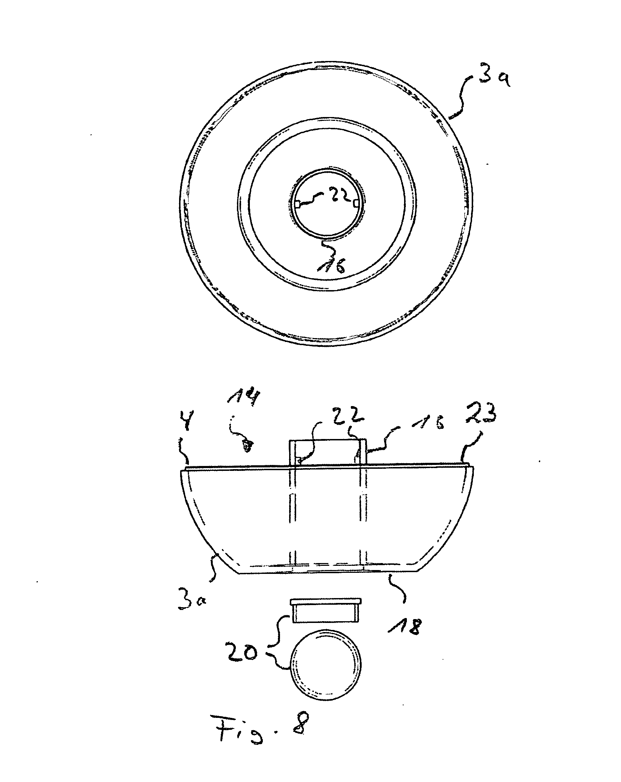

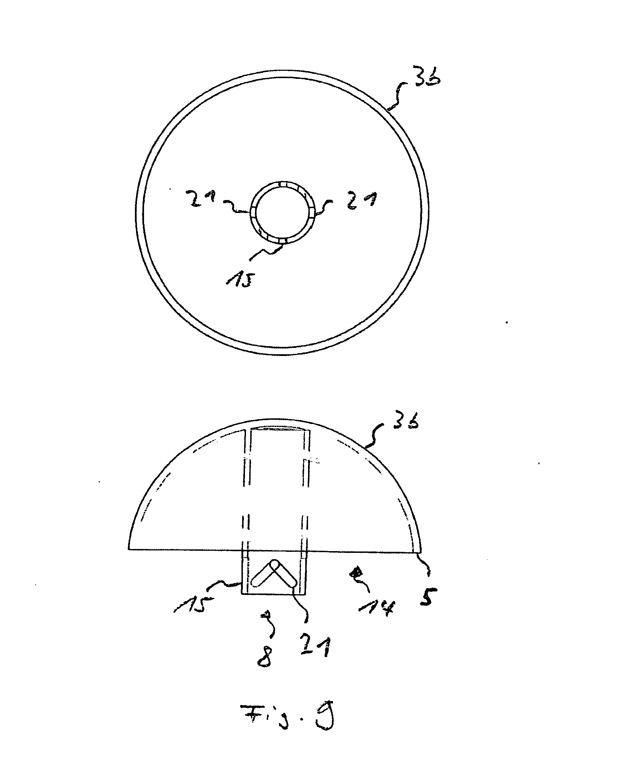

Es wird nun zusätzlich auf die Figuren 4 bis 10 Bezug genommen.

Um das Verlagern des Gehäuseoberteils 3b in Bezug zum Gehäuseunterteil 3a zu ermöglichen, sind das Gehäuseoberteil 3b und das Gehäuseunterteil 3a durch eine Gehäuseführung 8 miteinander verbunden. Die Gehäuseführung 8 ist in dem Gehäuseinneren 14 des Gehäuses 3, d. h. des Gehäuseunterteils 3a und des Gehäuseoberteils 3b angeordnet. Sie ist dabei zweigeteilt ausgebildet, d. h. die Gehäuseführung 8 weist einen ersten Abschnitt 15 auf, der dem Gehäuseoberteil 3b zugeordnet ist, und einen zweiten Abschnitt 16 auf, der dem Gehäuseunterteil 3a zugeordnet ist. Die beiden Abschnitte 15, 16 sind als zylinderartige, rohrförmige Abschnitte ausgebildet, wobei der dem Gehäuseunterteil 3a zugeordnete Abschnitt 16 einen größeren Durchmesser aufweist als der dem Gehäuseoberteil 3b zugeordnete Abschnitt 15, so dass sie zusammen eine teleskopartige Führung zur Verlagerung des Gehäuseoberteils 3b in Bezug zum Gehäuseunterteil 3a bilden. Zum Verschließen der unteren Öffnung des rohrförmigen Abschnitts 16 ist ein Verschlussstopfen 20 vorgesehen. Die Ausbildung des Abschnitts 16 als durchgehendes Hohlrohr erleichtert eine Entformung nach dem Spritzgießen des Gehäuseunterteils 3a.

Um eine besonders einfache Verstellung des Gehäuseoberteils 3b in Bezug zum Gehäuseunterteil 3a zu bewirken, ist die Gehäuseführung 8 derart ausgebildet, dass eine Drehbewegung D um die Verbindungsachse A eine Verbreiterung des Spalts 17 bewirkt.

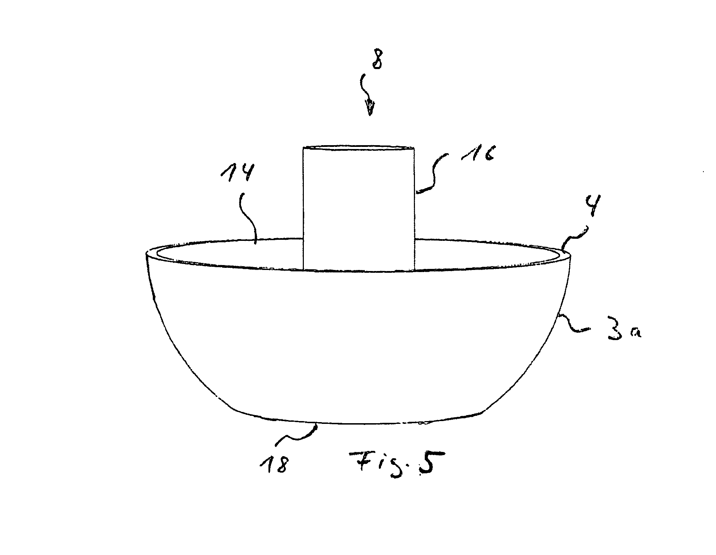

Hierzu ist in einem ersten Ausführungsbeispiel (vgl. Figuren 4 und 5) der dem Gehäuseoberteil 3b zugeordnete Abschnitt 15 mit einem Außengewinde 9 versehen, das in ein Innengewinde (nicht dargestellt) des Abschnitts 16 des Gehäuseunterteils 3a eingreift und so sicherstellt, dass durch Ausführen einer Drehbewegung D des Gehäuseoberteils 3b relativ zu dem Gehäuseunterteil 3a eine Bewegung des Gehäuseoberteils 3b aus der Ausgangsposition I in die zweite Position II möglich ist.

In einer zweiten Ausführungsform (vgl. Figuren 7, 8 und 9) ist der dem Gehäuseunterteil 3a zugeordnete Abschnitt 16 mit zwei Dornen 22 versehen, die in Eingriff mit als Führungskulissen für die Dorne 22 dienenden Schlitzen 21 bringbar sind, die in den rohrförmigen Abschnitt 15 eingebracht sind, die dem Gehäuseoberteil 3b zugeordnet sind. Durch eine entsprechende Anordnung der Schlitze 21, z. B. im Winkel von 45° zur Verbindungsachse A, wird erreicht, dass bei einer Drehbewegung D des Gehäuseoberteils 3b in Bezug zum Gehäuseunterteil 3a eine Drehbewegung D um die Verbindungsachse A in eine lineare Bewegung entlang der Verbindungsachse A bewirkt wird, mit der Folge, dass der Spalt 17 vergrößert wird.

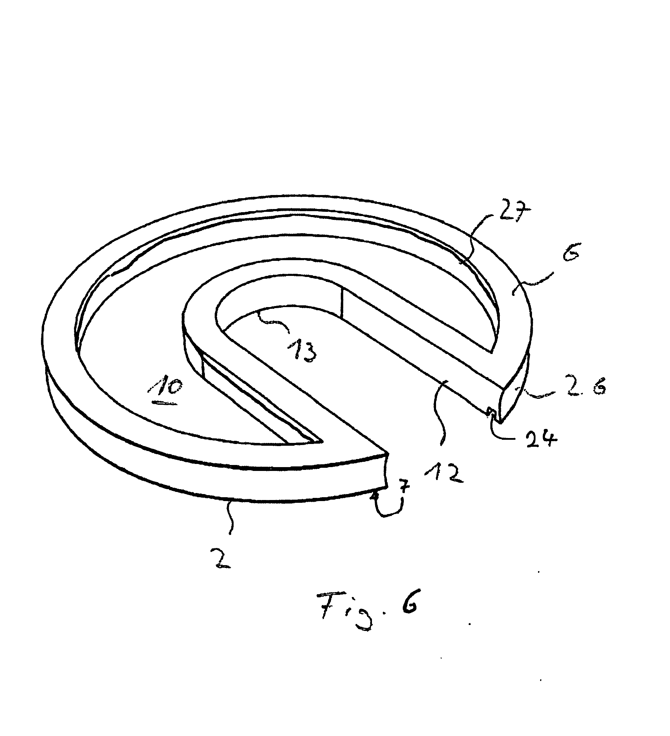

Der Wirkstoffträger 2 selber weist (siehe Figur 6) eine Kavität 10 auf, in der ein Trägerstoff 27, in z. B. gelförmiger Form aufgenommen ist, der einen gasförmigen Duftstoff als Wirkstoff freisetzt. Alternativ kann auch in der Kavität 10 ein Textil oder ein Vlies zur Aufnahme eines entsprechenden, auch flüssigen, Wirkstoffs angeordnet sein.

Der Wirkstoffträger 2 weist eine - von einer Ausnehmung 25 bzw. Öffnung oder Ausschnitt unterbrochene - kreisförmige Umrandung 26 auf, deren Radius gleich dem maximalen Radius des Gehäuseunterteils 3a und Gehäuseoberteils 3b ist. Die Ausnehmung 25 erstreckt sich radial bis über den Mittelpunkt des Wirkstoffträgers 2 in den Wirkstoffträgers 2 hinein und ist U-förmig ausgebildet, wobei die Breite b bzw. der Radius des U-Grunds der Ausnehmung 25 an den Außenradius des zweiten Abschnitts 16 der teleskopartigen Verbindungsachse bzw. Gehäuseführung 8 angepasst ist.

Der Wirkstoffträger 2 weist eine Wirkstoffträgerkontaktfläche 6 auf, der in der ersten Position I mit einer Gehäuseoberteilkontaktfläche 5 des Gehäuseoberteils 3b in Kontakt steht. Ferner weist der Wirkstoffträger 2 eine zweite Wirkstoffträgerkontaktfläche 7 auf, die mit einer Gehäuseunterteilkontaktfläche 4 des Gehäuseunterteils 3a in Kontakt steht. Dabei sind die Gehäusekontaktflächen 4 und 5 ringförmig ausgebildet, während die Wirkstoffträgerkontaktflächen 6, 7 als Ringsegmente ausgebildet sind.

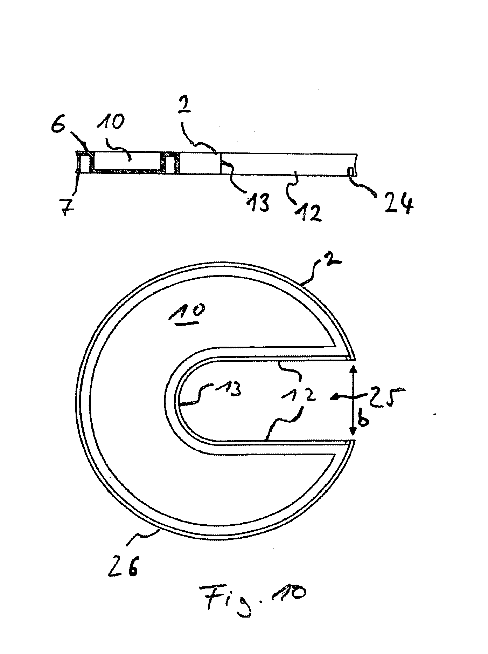

Zur Fixierung des Wirkstoffträgers 2 im Gehäuse 3 weist das Gehäuseunterteil 3a einen umlaufenden Haltering 23 auf, der in eine entsprechende Nut 24 des Wirkstoffträgers 2 (siehe Figur 10) eingreift und so den Wirkstoffträger 2 in seiner Position in Bezug zum Gehäuseunterteil 3a fixiert.

Im Bereich der Ausnehmung 25 des Wirkstoffträgers 2 sind zwei parallel zu einem Radius des Wirkstoffträgers 2 und parallel zueinander verlaufende Begrenzungsflächen als Wirkstoffträgerführung 12 ausgebildet. Der Abstand der Wirkstoffträgerführung 12 ist dabei so gewählt, dass er mit geringem Spiel über den Außendurchmesser des rohrförmigen Abstands 16 des Gehäuseunterteils 3a passt. Ferner weist der Wirkstoffträger 2 einen bogenförmig ausgebildeten Anschlag 13 - den oben genannten U-Grund -auf, dessen Radius wie erwähnt dem Radius des rohrförmigen Abschnitts 16 des Gehäuseunterteils 3a entsprechend angepasst ist. Somit wird der Wirkstoffträger 2 durch die Wirkstoffträgerführung 12 geführt und durch den Anschlag 13 blockiert.

Zur Inbetriebnahme der Wirkstoff-Abgabevorrichtung 1 wird bei einer Variante die Ausdampföffnung 19 geöffnet, z. B. indem ein selbstklebendes Verschlussmittel von der Ausdampföffnung 19 entfernt wird. Dadurch, dass das Gehäuse 3 mit dem Gehäuseunterteil 3a und dem Gehäuseoberteil 3b hohl ausgebildet ist, konnte sich schon vor Entfernen des Verschlusses der Ausdampföffnung 19 ein die freizusetzenden Wirkstoffe enthaltendes Gasgemisch bilden, das nun unmittelbar austritt und einem Benutzer im Fall von Duftstoffen ein besonders intensives Geruchserlebnis zu Beginn der Inbetriebnahme beschert.

Bei einer bevorzugten alternativen Variante befindet sich in einem Auslieferungszustand über der Kavität 10 des Wirkstoffträgers 2 eine gasundurchlässige Schutzfolie (nicht dargestellt), die zum Beispiel mit einer Oberseite der Umrandung 26 des Wirkstoffträgers 2 verklebt ist. Der Verbraucher kann dann den Wirkstoffträger 2 in der später erläuterten Weise aus dem Gehäuse 3 entnehmen, die Schutzfolie abziehen, und den Wirkstoffträger 2 wieder im Gehäuse 3 einsetzen.

Eine Regulierung des Austritts der Wirkstoffe, d. h. der Abdampfrate, kann in der Folge dadurch erfolgen, dass eine Drehbewegung D des Gehäuseoberteils 3b in Bezug zum Gehäuseunterteil 3a durchgeführt wird, wobei durch die Gehäuseführung 8 eine Umsetzung dieser Drehbewegung D um die Verbindungsachse A in eine lineare Bewegung entlang der Achse A des Gehäuseoberteils 3b in Bezug zum Gehäuseunterteil 3a bewirkt wird, mit der Folge, dass die Größe des Spaltes 19 vergrößert und die Ausdampföffnung 19' ringförmig erweitert ist, so dass nun Wirkstoffe mit einer höheren Rate austreten können.

Die Maße des Gehäuses 3 und des Wirkstoffträgers 2 sind bei den dargestellten bevorzugten Ausführungsbeispielen wie folgt: Der Durchmesser des Außen-Querschnitts des Gehäuses 3 und des Wirkstoffträgers 2 senkrecht zur Verbindungsachse A beträgt je nach Ausführungsbeispiel zwischen 7,5 cm und 8 cm. Die Höhe des Gehäuses 3 beträgt, bei einer Kugelform wegen der Abflachung zur Bildung der Standfläche 18, dann ca. 7,2 cm. Die Dicke (bzw. Höhe) des scheibenförmigen Wirkstoffträgers 2 ist so gewählt, dass eine Füllhöhe in der Kavität von ca. 4 mm gewährleistet ist. Die Breite des Spalts ist zwischen einem Minimalwert von ca. 5 mm und einem Maximalwert von ca. 10 mm bis ca. 15 mm einstellbar.

Wenn der Vorrat an Duftstoffen im Wirkstoffträger 2 erschöpft ist, kann ein Wechsel des Wirkstoffträgers 2 vorgenommen werden, indem der Wirkstoffträger 2 in Richtung der Verbindungsachse A angehoben wird, bis der Haltering 23 nicht mehr in die Nut 24 des Wirkstoffträgers 2 eingreift. Anschließend kann durch eine zweite Bewegung in eine Richtung senkrecht zur Verbindungsachse A, d. h. entlang der Montageachse B, der Wirkstoffträger aus dem Spalt 17 geschoben werden, wobei diese Bewegung durch das Zusammenwirken der Wirkstoffführung 12 und den beiden Abschnitten 15, 16 der Gehäuseführung 8 geführt wird. Nach Entfernen des verbrauchten Wirkstoffträgers 2 kann ein neuer unverbrauchter Wirkstoffträger 2 eingesetzt werden.

Dieser Wirkstoffträger 2 weist wieder eine Kavität 10 auf, in der ebenfalls ein Trägerstoff 27 angeordnet ist. Dabei ist die Kavität 10 wie oben erläutert mit einer Folie gasdicht verschlossen, so dass ein ungewünschter Austritt von Wirkstoffen vor der Inbetriebnahme ausgeschlossen ist. Vor dem Einsetzen des neuen Wirkstoffträgers 2 in die Wirkstoff-Abgabevorrichtung 1 wird diese Abdeckfolie von der Kavität 10 entfernt.

Anschließend wird der neue Wirkstoffträger 2 in den Spalt 17 der Wirkstoff-Abgabevorrichtung 1 eingeführt. Hierzu wird der Wirkstoffträger 2 entlang der senkrecht zur Verbindungsachse A verlaufenden Montageachse B derart bewegt, dass der erste oder zweite Abschnitt 15, 16 der Gehäuseführung 8 durch die Ausnehmung 25 des Wirkstoffträgers 2 hindurch tritt bzw. in die Ausnehmung 25 eingreift und mit der Führung 12 des Wirkstoffträgers 2 in Kontakt tritt. Der Wirkstoffträger 2 wird weiterbewegt, bis der erste oder zweite Abschnitt 15, 16 der Gehäuseführung 8 in Kontakt mit dem Anschlag 13 des Wirkstoffträgers 2 tritt. Anschließend erfolgt eine Bewegung in Richtung der Verbindungsachse A in Richtung zu dem Gehäuseunterteil 3a, um den Haltering 23 des Gehäuseunterteils 3a in Eingriff mit der Nut 24 des Wirkstoffträgers 2 zu bringen. Schließlich ist wieder eine Regulierung der Freisetzung des Wirkstoffes möglich, indem das Gehäuseoberteil 3b in Bezug zum Gehäuseunterteil 3a gedreht wird, um damit die Breite des Spaltes 17 entsprechend der gewünschten Abdampfrate anzupassen.

Alternativ kann der Austausch eines Wirkstoffträgers und das Einsetzen eines neuen Wirkstoffträgers dadurch erfolgen, dass das Gehäuseoberteil 3b vollständig vom Gehäuseunterteil 3a entfernt wird. Dies ist dann sinnvoll, wenn ein Wirkstoffträger verwendet werden soll, der anstelle der U-förmigen Ausnehmung 25 ein passendes zentrales Loch aufweist (nicht dargestellt). Entsprechend kann der Wirkstoffträger durch eine einfache Bewegung entlang der Verbindungsachse A entfernt und ein neuer Wirkstoffträger 2 wieder eingesetzt werden.

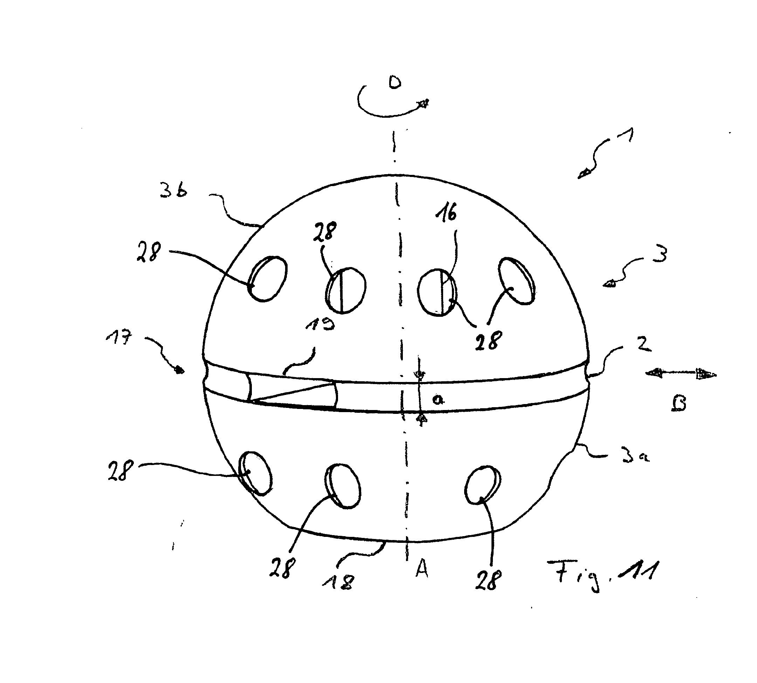

Figur 11 zeigt schließlich eine perspektivische Darstellung eines Wirkstoffträgers gemäß einem dritten Ausführungsbeispiel. Im Gehäuseoberteil 3a und im Gehäuseunterteil 3b sind hier Luftströmungslöcher 28, z.B. Bohrungen oder bei Spritzgießen des Teils mit eingebrachte Löcher, als weitere Ausdampföffnungen 28 angeordnet. Durch die im Gehäuseunterteil 3b angeordneten Luftströmungslöcher 28 und durch die Aussparung 25 im Wirkstoffträger 2 gebildete Ausdampföffnung 19 und/oder den Spalt 17 kann bei diesem Ausführungsbeispiel Luft in das Gehäuse 3 einströmen und am Wirkstoffträger 2 vorbei durch die weiter oben liegenden Luftströmungslöcher 28 beziehungsweise Ausdampföffnungen wieder ausströmen. Durch diesen Kamineffekt wird der Wirkstoff besonders gut verteilt.

In der Figur 12 ist ein alternativer Wirkstoffträger 2' dargestellt. Dieser weist eine Kavität 10 auf, in die ein mit dem Wirkstoff und gegebenenfalls dem Trägermaterial gefüllter Wirkstoffbehälter 30 gemäß Figur 13 eingesetzt werden kann. Wenn der Wirkstoff verbraucht ist, muss daher bei diesem Beispiel nicht der komplette Wirkstoffträger 2' ausgetauscht werden, sondern lediglich der Wirkstoffbehälter 30.

Figur 12 zeigt unten eine Draufsicht auf diesen alternativen Wirkstoffträger 2', und darüber zwei Schnittdarstellungen jeweils entlang der in der Draufsicht eingezeichneten Schnittlinie S₁, einmal ohne eingelegten Wirkstoffbehälter 30 (ganz oben) und einmal mit eingelegtem Wirkstoffbehälter 30 (mittlere Darstellung). Grundsätzlich ist dieser Wirkstoffträger 2' sehr ähnlich wie der Wirkstoffträger 2 gemäß den Figuren 6 und 10 aufgebaut. Insbesondere ist der Wirkstoffträger 2' ebenfalls im Wesentlichen scheibenförmig ausgebildet und weist eine äußere kreisförmige Umrandung 26 und eine nahezu identische U-förmigen Ausnehmung 25 wie der Wirkstoffträger 2 gemäß den Figuren 6 und 10 auf. Auch hier ist der Radius des U-Grunds der Ausnehmung 25 an den Außenradius des zweiten Abschnitts 16 der teleskopartigen Verbindungsachse bzw. Gehäuseführung 8 angepasst, so dass die Seiten der Ausnehmung 25 eine Wirkstoffträgerführung 12 und der U-Grund einen Anschlag 13 bilden, um den Wirkstoffträger 2' im Spalt 17 des Gehäuses 3 zu positionieren. Um jedoch mehr Platz für den Wirkstoff zur Verfügung zu stellen, ist die Kavität 10 hier tiefer als die Höhe des Wirkstoffträgers 2' an der äußeren Umrandung 26 ausgebildet. D.h. der Wirkstoffträger 2' ist im Bereich der Kavität 10 wie ein flacher, in der Draufsicht U-förmiger Topf ausgebildet, wobei die Wandungen dieses U-förmigen Topfs bzw. der Kavität 10 nach unten (axial bezüglich der Symmetrieachse der äußeren kreisförmigen Grundform des Wirkstoffträgers 2'), über die äußere Umrandung 26 hinaus ragt. Die Form des Topfs bzw. der Kavität 10 ist dabei so gewählt, dass dieser untere Teil des Wirkstoffträgers 2' in das Gehäuseunterteil 3a eingelegt werden kann und vorzugsweise darin passig gehalten wird. Die Tiefe der Kavität 10 ist hier vorzugsweise so gewählt, dass der gesamte Wirkstoffträger 2' in der oberen Stellung des Gehäuseoberteils 3a (siehe Figur 2) durch den erweiterten Spalt aus dem Gehäuse 3 herausgenommen bzw. in dieses eingesetzt werden kann.

An der Innenseite der radial äußeren Wand der Kavität 10 verläuft entlang zumindest eines Teils entlang des Umfangs eine Wulst 29. Diese wirkt mit einer Wulst 33 an einer Außenwand des Wirkstoffbehälters 30 zusammen, so dass dieser durch Klemmung in der Kavität 10 gehalten wird, beziehungsweise in die Kavität eingeclipst werden kann.

In Figur 13 ist hierzu eine Draufsicht und darüber eine Schnittdarstellung entlang der in der Draufsicht eingezeichneten Schnittlinie S₂ des passenden Wirkstoffbehälters 30 dargestellt. Wie aus diesen Figuren ersichtlich ist, ist der Wirkstoffbehälter 30 mit seinen äußeren Abmessungen an die Innenabmessungen der Kavität 10 des Wirkstoffträgers 2' angepasst, d.h. er ist in der Draufsicht im Wesentlichen U-förmig ausgebildet. Der Wirkstoffbehälter 30 besitzt ein oben offenes topfförmiges Gehäuse 31 zur Aufnahme des Wirkstoffs, ggf. mit einem beispielsweise flüssigen oder gelartigen Trägerstoff, auf, an dem oben seitlich ein rundum laufender Flansch 34 angeformt ist. Dieses Gehäuse 31 kann vorteilhaft kostengünstig aus Tiefziehfolie hergestellt sein, welche gerade noch so dick ist, dass das Gehäuse 31 unter normalen Temperaturbedingungen seine Form behält. Nach der Befüllung des Gehäuses 31 wird dieses mit einer vorzugsweise semipermeablen Membran 32 abgedeckt, welche am Rand mit dem Flansch 34 dicht verbunden, beispielsweise verklebt oder verschweißt wird. Zusätzlich kann diese Membran 32 zunächst noch mit einer Schutzfolie (nicht dargestellt) abgedeckt sein, welche vom Verbraucher abgezogen werden kann, wenn der Wirkstoffbehälter 30 in den Wirkstoffträger 2' eingesetzt worden ist und in Betrieb genommen werden soll.

Bei all den vorgeschriebenen Ausführungsbeispielen ist es möglich, in ein Gehäuse 3 statt nur eines Wirkstoffträgers 2 mehrere, vorzugsweise scheibenförmige, Wirkstoffträger 2 einzusetzen, die unterschiedliche Wirkstoffe, z. B. Wirkstoffe mit unterschiedlichen Duftnoten, freisetzen. Hierzu können die Wirkstoffträger 2 entlang der Verbindungsachse A übereinander stapelförmig angeordnet sein. Zur sicheren Fixierung der Wirkstoffträger 2 können diese z. B. auf ihrer Oberseite mit einem Haltering versehen sein und auf ihrer Unterseite mit einer entsprechenden Nut 24. Insbesondere können dabei auch Wirkstoffträger 2, 2' unterschiedlicher Ausbildungen kombiniert werden, beispielsweise zuunterst ein Wirkstoffträger 2' gemäß Figur 12 und darüber ein oder mehrere Wirkstoffträger 2 gemäß Figur 10 oder umgekehrt. Um einen simultanen Austritt der verschiedenen Wirkstoffe zu ermöglichen, können ferner diese Wirkstoffträger 2 mit entsprechenden mehreren Öffnungen, Ausnehmungen oder Ausbrüchen versehen sein. Alternativ ist eine Anordnung mehrerer Wirkstoffträger 2 zueinander denkbar, bei der die Ausnehmungen 25 in Bezug auf die Verbindungsachse A mit einem Winkelversatz zueinander angeordnet ist, um so dreieckige bzw. tortenstückförmige Austrittsflächen für die Wirkstoffe bereitzustellen.

Somit wird zusammenfassend eine Wirkstoff-Abgabevorrichtung zur Abgabe von gasförmigen Stoffen, insbesondere Duftstoffen, bereitgestellt, die auf einfache Art und Weise eine Regulierung der Freisetzrate der Wirkstoffe erlaubt und zugleich einen besonders einfachen Ersatz eines Wirkstoffträgers nach Erschöpfung erlaubt.

Es wird abschließend noch einmal darauf hingewiesen, dass es sich bei den in den Figuren dargestellten Wirkstoff-Abgabevorrichtungen, Gehäusen und Wirkstoffträgern nur um Ausführungsbeispiele handelt, welche vom Fachmann in vielfacher Hinsicht variiert werden können, ohne den Rahmen der Erfindung zu verlassen. So sind insbesondere auch verschiedenste Kombinationen der gezeigten Bestandteile der Wirkstoff-Abgabevorrichtung möglich. Es wird außerdem der Vollständigkeit halber darauf hingewiesen, dass die Verwendung des unbestimmten Artikels "ein" bzw. "eine" nicht ausschließt, dass die betreffenden Merkmale auch mehrfach vorhanden sein können.

### Bezugszeichenliste

- 1: Wirkstoff-Abgabevorrichtung
- 2, 2': Wirkstoffträger
- 3: Gehäuse
- 3a: Gehäuseunterteil
- 3b: Gehäuseoberteil
- 4: Gehäuseunterteilkontaktfläche
- 5: Gehäuseoberteilkontaktfläche
- 6: Wirkstoffträgerkontaktfläche
- 7: Wirkstoffträgerkontaktfläche
- 8: Gehäuseführung
- 9: Außengewinde
- 10: Kavität
- 12: Wirkstoffträgerführung
- 13: Anschlag
- 14: Gehäuseinneres
- 15: erster Abschnitt
- 16: zweiter Abschnitt
- 17: Spalt
- 18: Standfläche
- 19: Ausdampföffnung / Luftströmungsloch
- 19': vergrößerte Ausdampföffnung
- 20: Verschlussstopfen
- 21: Schlitz
- 22: Dorn
- 23: Haltering
- 24: Nut
- 25: Ausnehmung
- 26: Umrandung
- 27: Trägerstoff
- 28: Ausdampföffnung / Luftströmungsloch29 Halteelement / Wulst
- 30: Wirkstoffbehälter
- 31: Gehäuse
- 32: semipermeable Membran
- 33: Halteelement / Wulst
- 34: Flansch
- a: Abstand
- a': vergrößerter Abstand
- b: Breite
- A: Verbindungsachse
- B: Montageachse
- D: Drehbewegung
- S₁: Schnittlinie
- S₂: Schnittlinie

## Patentansprüche

1. Wirkstoff-Abgabevorrichtung (1) zur Abgabe von gasförmigen Wirkstoffen, insbesondere von Duftstoffen, in die Umgebung, mit
- einem Gehäuse (3) und
- einem dem Gehäuse (3) zugeordneten Wirkstoffträger (2, 2'),
wobei das Gehäuse (3) ein Gehäuseunterteil (3a) und ein Gehäuseoberteil (3b) umfasst und wobei das Gehäuseoberteil (3b) und das Gehäuseunterteil (3a) unter Bildung eines Spalts (17) mit einem Abstand (a) voneinander angeordnet sind, und in dem Spalt (17) zumindest teilweise der Wirkstoffträger (2, 2') angeordnet ist.

2. Wirkstoff-Abgabevorrichtung (1) nach Anspruch 1, **dadurch gekennzeichnet, dass** das Gehäuseoberteil (3b) eine Gehäuseoberteilkontaktfläche (5) aufweist, der an eine erste Wirkstoffträgerkontaktfläche (6) des Wirkstoffträgers (2, 2') angrenzt, und das Gehäuseunterteil (3a) wenigstens eine Gehäuseoberteilkontaktfläche (4) aufweist, die an eine zweite Wirkstoffträgerkontaktfläche (7) des Wirkstoffträgers (2, 2') angrenzt.

3. Wirkstoff-Abgabevorrichtung (1) nach Anspruch 1 oder 2, **dadurch gekennzeichnet, dass** der Abstand (a, a') vom Gehäuseoberteil (3b) zu dem Gehäuseunterteil (3a) durch Verlagern des Gehäuseoberteils (3b) relativ zum Gehäuseunterteil (3a) entlang einer Verbindungsachse (A) variierbar ist.

4. Wirkstoff-Abgabevorrichtung (1) nach Anspruch 3, **gekennzeichnet durch** eine Gehäuseführung (8), **durch** die das Gehäuseunterteil (3a) und das Gehäuseoberteil (3b) so gekoppelt sind, dass zum Verlagern eine lineare Bewegung des Gehäuseoberteils (3b) relativ zum Gehäuseunterteil (3a) entlang der Verbindungsachse (A) durchführbar ist.

5. Wirkstoff-Abgabevorrichtung (1) nach einem der Ansprüche 3 oder 4, **dadurch gekennzeichnet, dass** der Wirkstoffträger (2, 2') durch ein Verlagern des Wirkstoffträger (2, 2') relativ zum Gehäuse (3) quer zur Verbindungsachse (A) in oder aus dem Spalt (17) bringbar ist.

6. Wirkstoff-Abgabevorrichtung (1) nach einem der Ansprüche 1 bis 5, **dadurch gekennzeichnet, dass** der Wirkstoffträger (2, 2') eine Kavität (10) zur Aufnahme des Wirkstoffs und gegebenenfalls eines den Wirkstoff enthaltenden Trägerstoffs (27) und/oder zur Aufnahme eines Wirkstoffbehälters (30) aufweist, welcher den Wirkstoff und gegebenenfalls den Wirkstoff enthaltenden Trägerstoff enthält.

7. Wirkstoff-Abgabevorrichtung (1) nach einem der Ansprüche 1 bis 6, **dadurch gekennzeichnet, dass** die Kavität (10) im Wirkstoffträger (2, 2') und/oder dass der Wirkstoffbehälter (30) durch eine semipermeable Membran abgedeckt ist und/oder dass sich in der Kavität (10) und/ oder dem Wirkstoffbehälter (30) ein gelartiger Trägerstoff (27) mit dem Wirkstoff befindet.

8. Wirkstoff-Abgabevorrichtung (1) nach einem der Ansprüche 1 bis 7, **dadurch gekennzeichnet, dass** das Gehäuseunterteil (3a) und das Gehäuseoberteil (3b) jeweils angrenzend an den Spalt (17) senkrecht zur Längsrichtung der Verbindungsachse (A) einen kreisförmigen Außen-Querschnitt aufweisen.

9. Wirkstoff-Abgabevorrichtung (1) nach einem der Ansprüche 4 bis 8, **dadurch gekennzeichnet, dass** der Wirkstoffträger (2, 2') eine Wirkstoffträgerführung (12) aufweist, um den Wirkstoffträger (2, 2') beim Einschieben in den Spalt (17) zu führen, und der Wirkstoffträger (2, 2') wenigstens einen Anschlag (13) aufweist, um eine Bewegung in den Spalt (17) zu begrenzen, wobei die Wirkstoffträgerführung (12) und/oder der Anschlag (13) mit der Gehäuseführung (8) des Gehäuses (3) zusammenwirken.

10. Wirkstoff-Abgabevorrichtung (1) nach einem der Ansprüche 4 bis 9, **dadurch gekennzeichnet, dass** die Gehäuseführung (8) im Inneren (14) des Gehäuses (3) angeordnet ist.

11. Wirkstoff-Abgabevorrichtung (1) nach einem der Ansprüche 1 bis 10, **dadurch gekennzeichnet, dass** das Gehäuseunterteil (3a) und/oder das Gehäuseoberteil (3b) und/oder der Wirkstoffträger (2, 2') eine Anzahl von Luftströmungslöchern (19, 28) aufweisen.

12. Wirkstoff-Abgabevorrichtung (1) nach Ansprüche 4 bis 11, **dadurch gekennzeichnet, dass** die Gehäuseführung (8) einen ersten Abschnitt (15) und einen zweiten Abschnitt (16) umfasst, wobei der erste und/oder zweite Abschnitt (15, 16) sich in Richtung der Verbindungsachse (A) aus dem Gehäuseunterteil (3a) und/oder Gehäuseoberteil (3b) erstrecken.

13. Gehäuse (3) für eine Wirkstoff-Abgabevorrichtung (1) nach einem der Ansprüche 1 bis 12.

14. Wirkstoffträger (2, 2') für eine Wirkstoff-Abgabevorrichtung (1) nach einem der Ansprüche 1 bis 12.

15. Wirkstoffbehälter (30) enthaltend einen Wirkstoff, wobei der Wirkstoffbehälter (30) zur Aufnahme in einem Wirkstoffträger (2') nach Anspruch 14 ausgebildet ist.
